(19) Europäisches Patentamt
European Patent Office
Office européen des brevets

(11) Veröffentlichungsnummer: **0 205 890 B1**

(12) **EUROPÄISCHE PATENTSCHRIFT**

(45) Veröffentlichungstag der Patentschrift: **15.07.92**

(51) Int. Cl.⁵: **C08G 77/52**, C08G 77/58, C12N 11/00

(21) Anmeldenummer: **86106593.6**

(22) Anmeldetag: **15.05.86**

# (54) Phenylengruppen-haltige Organopolysiloxane und Verfahren zu ihrer Herstellung.

(30) Priorität: **25.05.85 DE 3518879**

(43) Veröffentlichungstag der Anmeldung:
**30.12.86 Patentblatt 86/52**

(45) Bekanntmachung des Hinweises auf die Patenterteilung:
**15.07.92 Patentblatt 92/29**

(84) Benannte Vertragsstaaten:
**AT BE CH DE FR GB IT LI NL SE**

(56) Entgegenhaltungen:
**GB-A- 915 931**
**US-A- 3 304 320**
**US-A- 4 361 691**
**US-A- 4 476 291**

(73) Patentinhaber: **Degussa Aktiengesellschaft**
**Weissfrauenstrasse 9**
**W-6000 Frankturt am Main 1(DE)**

(72) Erfinder: **Panster, Peter, Dr.**
**Im Lochseif 8**
**W-6458 Rodenbach(DE)**
Erfinder: **Kleinschmit, Peter, Dr.**
**Wildaustrasse 19**
**W-6450 Hanau 9(DE)**

**EP 0 205 890 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue Phenylengruppen-haltige Organopolysiloxane, die zu Trägern von Wirkstoffen funktionalisiert werden können und ein Verfahren zur Herstellung dieser Organopolysiloxane.

Wirkstoffe oder funktionelle Gruppen, die über chemische Bindungen an einen unlöslichen Träger gebunden sind, besitzen beim technischen Einsatz gegenüber in homogener Phase eingesetzten Wirkstoffen oder funktionellen Gruppen die möglichen Vorteile einer leichteren Abtrennbarkeit, Recyclierbarkeit und Rückgewinnbarkeit der aktiven Komponente. Darüber hinaus können Stabilität und Standzeit eines nach diesem Prinzip modifizierten Agens oft deutlich erhöht und seine Selektivität in einem gewünschten Sinne beeinflußt werden. Während z. B. Ionenaustauscher bereits klassische Beispiele für dieses Konzept darstellen, sind z.B. Trägerfixierte Enzyme oder Metallkomplexkatalysatoren Gegenstand jüngerer Untersuchungen und Synthesebemühungen.

Als Träger eingesetzt wurden für diesen Zweck bisher überwiegend organische Polymere, insbesondere Polystyrol. Beispiele hierfür werden z. B. in der britischen Patentschrift 1 277 736 oder in der US-Patentschrift 3 708 462 gegeben. Obwohl anorganische Polymersysteme, wie z. B. Kieselsäure oder Kieselgel, eine Reihe von Vorteilen besitzen, sind sie im allgemeinen für diese Verwendung weniger geeignet, denn sie sind nur in geringem Umfang funktionalisierbar und die funktionelle Einheit kann hydrolytisch relativ leicht abgespalten werden.

In Ermangelung wirklich geeigneter Trägersysteme wurde auch schon versucht, Phenylsiloxane auf Silicagel zu verankern (vgl. J.Conan, M.Bartholin und A.Guyet, J. Mol. Catal. 1, 375 (1975/76). Derartige Systeme besitzen im Prinzip aber dieselben Nachteile wie reine anorganische Träger selbst.

Aufgabe der vorliegenden Erfindung war es daher, Trägersysteme bereitzustellen, die sowohl die Vorteile der organischen als auch die der anorganischen Trägermaterialien in sich vereinigen, d. h. daß sie eine festliegende starre Struktur, hohe Temperatur- sowie Alterungsbeständigkeit besitzen, daß sie nur wenig oder gar nicht quellbar und unlöslich in organischen Lösungsmitteln sind und durch Einführung funktioneller Gruppen in Träger für Wirkstoffe verarbeitbar sind.

Die Aufgabe wird durch die Entwicklung neuer Phenylengruppen-haltiger Organopolysiloxane gelöst, die an den Phenylengruppen leicht nach bekannten Konzepten der organischen Synthese funktionalisiert und in gewünschter Weise und Umfang zu einem Wirkstoff- oder Wirkgruppenträger modifiziert werden können. Die neuen Phenylengruppen-haltigen Organopolysiloxane sind dadurch gekennzeichnet, daß sie gleiche oder verschiedene Einheiten der allgemeinen Formel

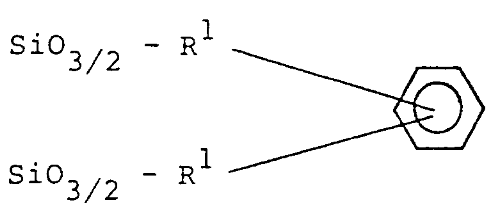

(1)

enthalten, in der jeweils alle 3 denkbaren Isomeren in bezug auf die Stellung der $SiO_{3/2}$-$R^1$-Substituenten an der Phenylengruppe nebeneinander vorliegen können, wobei die Brückenglieder $R^2$ für die Gruppen $-CH_2-CH_2-$ oder

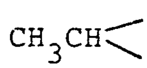

stehen und gleich oder verschieden sein können, und die freien Valenzen der Sauerstoffatome durch Siliciumatome weiterer Gruppen der Formel (1) und/oder durch vernetzende Brückenglieder

$SiO_{4/2}$ oder $R'SiO_{3/2}$ oder $R'_2\,SiO_{2/2}$,
$TiO_{4/2}$ oder $R'TiO_{3/2}$ oder $R'_2\,TiO_{2/2}$,
$ZrO_{4/2}$ oder $R'ZrO_{3/2}$ oder $R'_2\,ZrO_{2/2}$,
$AlO_{3/2}$ oder $R'AlO_{2/2}$

wobei R' eine Methyl- oder Ethylgruppe ist, abgesättigt sind und das Verhältnis der Summe der Si-Atome in Formel (1) zu den Brückenatomen Silicium,Titan, Zirkonium und Aluminium 1 : 0 bis 1 : 15 betragen kann.

Die Stellung der beiden $SiO_{3/2}$-$R^1$-Substituenten zueinander an der Phenylengruppe ist generell von

untergeordneter Bedeutung; es kann sowohl eine ortho-, als auch eine meta-, als auch eine para-Stellung gegeben sein. Im allgemeinen ist es im Hinblick auf eine möglichst hohe Kapazität an später an den Phenylengruppen zu fixierenden funktionellen Gruppen oder Wirkstoffgruppen wünschenswert, wenn keine vernetzenden Brückenglieder der genannten Art im Polymerverband vorhanden sind, weil sie keinen Beitrag zur Funktionalität leisten.

In verschiedenen Fällen, z. B. bei Verwendung der erfindungsgemäßen Polysiloxane als Träger von heterogenierten Komplexkatalysatoren, kann es jedoch von Vorteil sein, z.B. zum Zwecke der Steuerung der Wirkstoffgruppendichte oder zur Beeinflussung und Einstellung bestimmter spezifischer Oberflächen oder Porositäten oder auch bestimmter sterischer Verhältnisse oder Oberflächeneigenschaften vernetzende Brückenglieder der genannten Art in das Polymergerüst einzubauen. Daneben kann die Anwesenheit der Vernetzer z. B. auch aufgrund etwa gegebener katalytischen Eigenschaften derselben von Interesse sein.

Gegenstand der Erfindung ist auch ein Verfahren zur Herstellung der neuen Phenylengruppen-haltigen Organopolysiloxane. Es ist dadurch gekennzeichnet, daß ein Silan der allgemeinen Formel

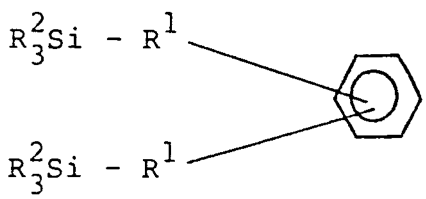

(2)

in der die Brückenglieder $R^1$ für die Gruppen $-CH_2-CH_2-$ oder

$$CH_3-CH<$$

stehen und gleich oder verschieden sein können, und die Substituenten $R^2$ für einen linearen oder verzweigten Alkoxirest mit 1 bis 3 C-Atomen oder für Chlorid stehen und gleich oder verschieden sein können , gegebenenfalls nach Zusatz eines Lösungsmittels und/oder einer Vernetzervorstufe der allgemeinen Formel

$$MeR^3_{2-4}R'_{0-2} \quad \text{bzw.} \quad MeR^3_{2-3}R'_{0-1}$$

wobei Me = Si, Ti, Zr bzw. Al
$R^3$ für einen linearen oder verzweigten Alkoxirest mit 1 bis 5 C-Atomen oder für Chlorid steht und
R' eine Methyl- oder Ethylgruppe ist, mit stöchiometrischen oder überschüssigen Mengen Wasser hydrolysiert und polykondensiert, das Produkt gegebenenfalls nach Zusatz weiteren Lösungsmittels von der flüssigen Phase abgetrennt, gegebenenfalls gewaschen, gegebenenfalls unter Schutzgasatmosphäre oder unter Verwendung von Vakuum bis zu einer Temperatur von 200° C getrocknet, anschließend gegebenenfalls 1 Stunde bis zu 5 Tage bei Temperaturen von 100 - 400° C an der Luft oder unter Schutzgas, bei Normaldruck, unter Vakuum oder Überdruck getempert, gegebenenfalls gemahlen und klassifiziert wird.

Bezüglich der Stabilität der neuen Phenylengruppen-haltigen Organopolysiloxane gegenüber An- oder Auflösen bei erhöhter Temperatur in Wasser oder aggressiven polaren organischen Solvenzien ist es von Vorteil, wenn das Produkt nach seiner Herstellung, gegebenenfalls in Verbindung mit einer Trocknung oder auch erst vor seiner Verwendung der erwähnten Temperung ausgesetzt wird. Die Maßnahme der Temperung ist von der Synthese von anorganischen Polymeren, wie z. B. Kieselsäuren oder Kieselgelen, her bekannt. Sie bewirkt eine weitere Dehydratisierung bei Reaktion benachbarter Silanolgruppen oder eine Abspaltung von noch in der polymeren Substanz vorhandenen Alkoxigruppen bzw. Si-gebundenen Chloratomen in Gestalt des entsprechenden Alkohols bzw. von Chlorwasserstoff bei gleichzeitiger Ausbildung von Siloxanbindungen.

Im Prinzip könnte $R^2$ noch für weitere Substituenten, wie z. B. Br, J, $OC_6H_5$ oder $OC_2H_4OCH_3$, stehen, doch bietet deren Verwendung keine Vorteile, sondern eher Nachteile, z. B. hinsichtlich der Zugänglichkeit der entsprechenden Silane oder in bezug auf die Hydrolysegeschwindigkeit und die bei der Hydrolyse

anfallenden Nebenprodukte. Verschiedentlich, z. T. in Abhängigkeit von der Art des verwendeten Lösungsvermittlers und wenn $R^2$ für einen linearen oder verzweigten Alkoxirest steht, kann es von Vorteil sein, dem zu polykondensierenden Silan eine kleine Menge eines der üblichen Polykondensationskatalysatoren, im einfachsten Fall wäßrige HCl-Lösung, zuzusetzen. Unter diesem Aspekt betrachtet, ist verständlicherweise die Hydrolysegeschwindigkeit am größten, wenn $R^2$ für Chlorid steht.

Obwohl die Hydrolyse und Polykondensation auch ohne Verwendung eines Lösungsvermittlers durchführbar ist, ist eine Benutzung aus praktischen Gründen meist vorzuziehen.

Als Lösungsmittel bieten sich insbesondere, wenn $R^2$ für einen linearen oder verzweigten Alkoxirest mit 1 bis 3 C-Atomen steht, die zu den Alkoxiresten korrespondierenden Alkohole an. Allerdings können auch andere Solvenzien, die keine Reaktion mit dem Silan eingehen, wie z. B. Toluol, Xylol, chlorierte Kohlenwasserstoffe, Nitromethan, Nitrobenzol, Aceton, Methylethylketon, Diethlketon, n-und i-Butanol, n-Pentanol, Diethylether, Di-n-propylether, Di-i-propylether, Di-n-butylether, Methyl-t-butylether, aliphatische, lineare, verzweigte oder cyclische Kohlenwasserstoffe, Dimethylsulfoxid, Dimethylformamid, verwendet werden.

Bei Zusatz einer Vernetzervorstufe ist natürlich die zu verwendende Mindestmenge Wasser, d. h. die stöchiometrische Menge entsprechend anzupassen. Natürlich kann die Hydrolyse und Polykondensation nicht nur bei Normaldruck, sondern auch unter Unterdruck oder unter Überdruck durchgeführt werden.

Die Abtrennung des Produkts von der flüssigen Phase kann nach gängigen Techniken, entweder durch Abdestillieren der Flüssigkeit oder durch Abfiltrieren oder Abzentrifugieren des Feststoffs erfolgen.

Die erfindungsgemäßen Organopolysiloxane besitzen je nach Ausgangsmaterial, verwendeten Polykondensationsmedium und Polykondensationsbedingungen spezifische Oberflächen von weniger als 1 $m^2/g$ bis zu 1000 $m^2/g$. Die Teilchengrößen können innerhalb bestimmter Bereiche eingestellt werden; sie liegen zwischen ca. 0,1 µm bis zu 1 cm.

Ein Gegenstand der Erfindung ist auch die Verwendung der neuen Phenylengruppen-haltigen Organopolysiloxane zur Synthese von Wirkstoffträgern durch Substitution mindestens einer der ringständigen Wasserstoffatome durch Trägerfunktion vermittelnde Substituenten nach üblichen Methoden der Organischen Chemie.

Die Erfindung wird nachfolgend anhand von Ausführungsbeispielen unter Verwendung der wichtigsten Ausgangsmaterialien näher erläutert.

Beispiel 1

100 g einer isomeren Verbindung, bestehend zu ca. 90 Gew.% aus dem ortho-, meta-, para-Isomerengemisch (12 Gew.%/ 65 Gew.%/ 23 Gew.%) des Chlorsilans

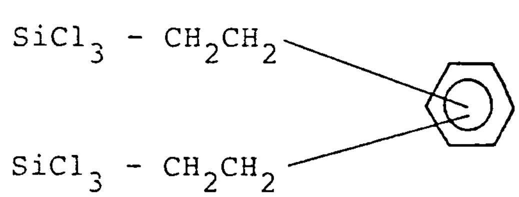

und zu ca. 10 % aus den Chlorsilanen

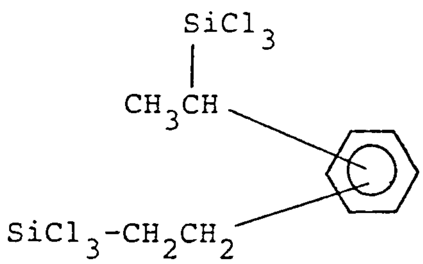

und

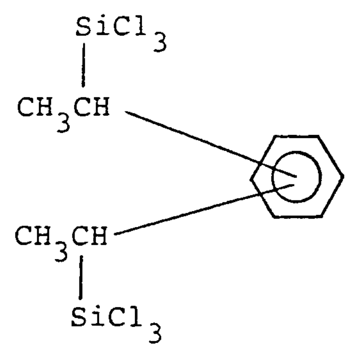

(gewichtsmäßige Mengenverteilung 2 : 1)

mit der gleichen Isomerenverteilung wurden in rund 100 ml Toluol gelöst. Die Lösung wurde in einem 1 l-Dreihalskolben mit KPG-Rührer und Rückflußkühler unter kräftigem Rühren und zunächst unter Eiskühlung innerhalb von 30 min. mit 100 g entsalztem Wasser versetzt. Unter starkem Schäumen setzte bereits nach Zusatz von 30 ml $H_2O$ eine spontane Verdickung ein, so daß der Kolbeninhalt kurzfristig nicht mehr rührfähig war. Nach Zugabe von weiteren 50 ml Toluol und 50 ml Wasser wurde auf Rückflußtemperatur erhitzt und 2 Stunden unter Rückfluß gerührt. Anschließend wurde abgekühlt und der gebildete weiße Feststoff über eine Nutsche abfiltriert, zunächst mit 100 ml Ethanol und dann mit 3 1 Wasser nahezu HCl-frei gewaschen. Nach 12-stündiger Trocknung bei 150° C/100 mbar und 30-stündiger Temperung bei 250° C unter $N_2$-Atmosphäre konnten 58,8 g (99,8 % der Theorie) des gewünschten Phenylengruppen-haltigen Organopolysiloxans, bestehend zu ca. 90 % aus Einheiten der Formel

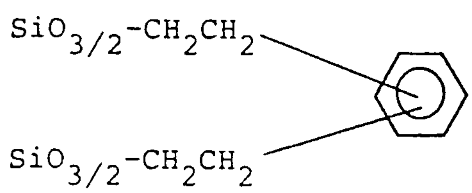

und zu ca. 10 % aus Einheiten der Formel

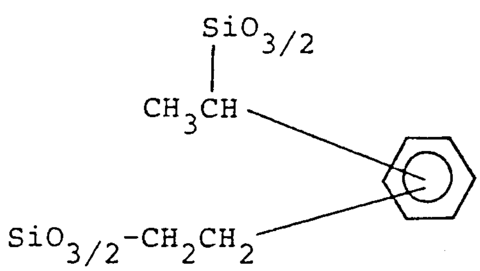

und

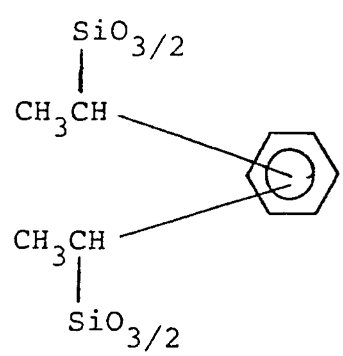

(entsprechend der im Ausgangsmaterial vorgegebenen gewichtsmäßigen Mengenverteilung)

in Form eines weißen Pulvers erhalten werden.

| Elementaranalysen: | % C | % H | % Si | % Cl |
|---|---|---|---|---|
| Theorie: | 50,81 | 5,12 | 23,76 | 0 |
| Gefunden: | 48,95 | 5,33 | 22,47 | 0,02 |

Das Produkt wurde nach der Trocknung und Temperung gemahlen und klassifiziert. Von der Korngrößenfraktion 0,3 - 1,2 mm wurde eine Bestimmung der spezifischen Oberfläche am Areameter durchgeführt, die einen Wert von 338 $m^2/g$ ergab. Eine durchgeführte DSC-Untersuchung des Produkts unter $N_2$-Atmosphäre ergab eine beginnende endotherme Zersetzung des Polymers bei über 280° C.

Beispiel 2

100 g einer isomeren Verbindung, bestehend zu ca. 90 % aus dem ortho-, meta-, para-Isomerengemisch (12 Gew.% / 65 Gew.% / 23 Gew..%) des Ethoxisilans

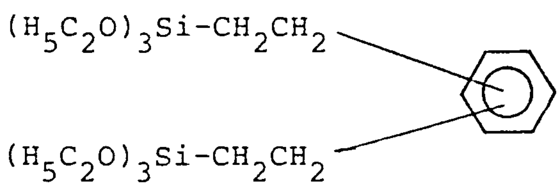

und zu ca. 10 % aus den Ethoxisilanen

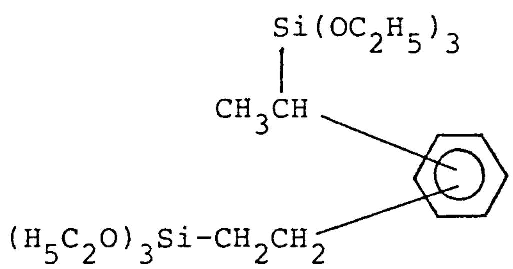

und

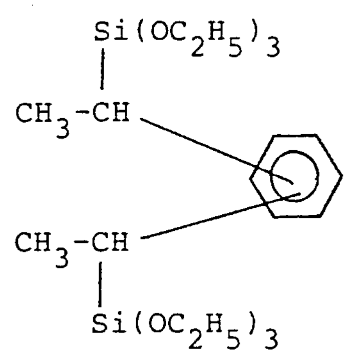

(gewichtsmäßige Mengenverteilung 2 : 1)

mit der gleichen Isomerenverteilung wurden mit 120 ml Ethanol gemischt. Die Mischung wurde in einem 1 l-Dreihalskolben mit KPG-Rührer, Rückflußkühler und Tropftrichter auf Rückflußtemperatur aufgeheizt und unter kräftigem Rühren auf einmal mit 50 ml $H_2O$ versetzt. Schon wenige Minuten nach der Wasserzugabe verdickte sich der Ansatz und es entstand ein voluminöser Feststoff. Dieser wurde noch 2 Stunden unter Rückfluß gerührt, dann über ein Saugfilter abfiltriert und erst mit 100 ml Ethanol und dann mit 2 1 $H_2O$ gewaschen. Nach 24-stündiger Trocknung bei 150° C/100 mbar wurden 52,7 g (102,3 % der Theorie) des gewünschten Produkts in Form eines weißen Feststoffs erhalten. Die Zusammensetzung dieses Organopolysiloxans entsprach der des in Beispiel 1 erhaltenen Produkts, sowohl in bezug auf die Struktur als auch hinsichtlich der Isomerenverteilung.

| Elementaranalysen: | % C | % H | % Si |
|---|---|---|---|
| Theorie: | 50,81 | 5,12 | 23,76 |
| Gefunden: | 49,03 | 6,01 | 22,34 |

Die 0,3 - 1,2 mm Fraktion des klassifizierten Produkts besaß eine spezifische Oberfläche von 89 $m^2/g$ (Areameter).

Beispiel 3

75 g des in Beispiel 2 eingesetzten Ausgangsmaterials und 68,12 g $Si(OC_2H_5)_4$ wurden in 100 ml Ethanol vereinigt. Die Mischung wurde in einem 1 l-Dreihalskolben mit KPG-Rührer, Rückflußkühler und Tropftrichter auf Rückflußtemperatur erhitzt. Unter kräftigem Rühren wurden auf einmal 50 g Wasser zugesetzt. Der Kolbeninhalt gelierte sofort nach Zugabe des Wassers. Der Kolbeninhalt wurde noch eine Stunde unter Rückfluß gerührt, dann abgekühlt, abfiltriert und mit 300 ml Ethanol gewaschen. Nach 10-stündiger Trocknung bei 150° C und 2-Stündiger Temperung bei 300° C unter $N_2$-Atmosphäre wurden 58,9 g (101,0 % der Theorie) eines polymeren Produkts, bestehend zu ca. 90 % aus Einheiten der Formel

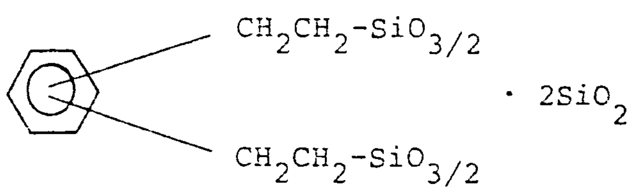

und zu ca. 10 % aus Einheiten der Formel

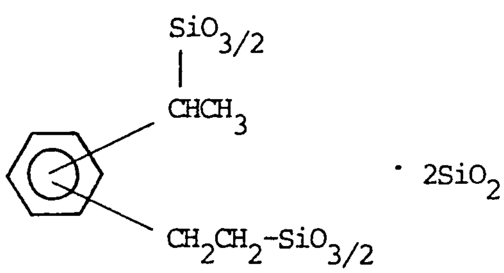

und

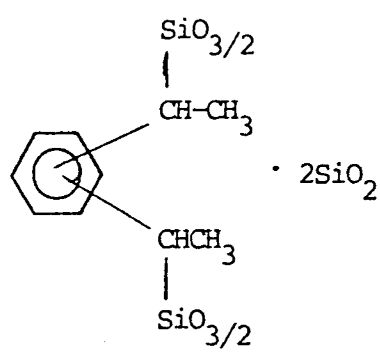

(entsprechend der im Ausgangsmaterial vorgegebenen gewichtsmäßigen Mengenverteilung)

mit einem ortho-/meta-/para-Isomerenverhältnis von 12 Gew.% / 65 Gew.% / 23 Gew.% erhalten.

| Elementaranalysen: | % C | % H | % Si |
|---|---|---|---|
| Theorie: | 33,69 | 3,39 | 31,51 |
| Gefunden: | 32,21 | 3,56 | 30,87 |

Beispiel 4

75 g einer isomeren Verbindung, bestehend zu rund 100 % aus dem meta- und para-Isomerengemisch (60 Gew.% / 40 Gew.%) des Methoxisilans

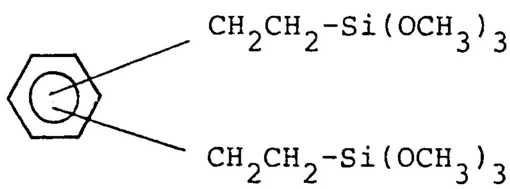

und 29,7 g $(H_3C)_2Si(OC_2H_5)_2$ wurden in 100 ml Aceton vereinigt. Die Mischung wurde in einem 1 l-Dreihalskolben mit KPG-Rührer, Rückflußkühler und Tropftrichter auf Rückflußtemperatur erhitzt. Dann wurden 40 g Wasser auf einmal zugesetzt. Nach kurzer Zeit bildete sich im Kolben ein voluminöser Feststoff, der noch eine Stunde unter Rückfluß gerührt, dann abzentrifugiert, mit 250 ml Aceton gewaschen und 15 Stunden bei 150° C getrocknet. Nach 24-stündiger Temperung bei 250° C unter $N_2$-Atmosphäre wurden 61,9 g (99,6 % der Theorie) des gewünschten polymeren Produkts, bestehend aus Einheiten der Formel

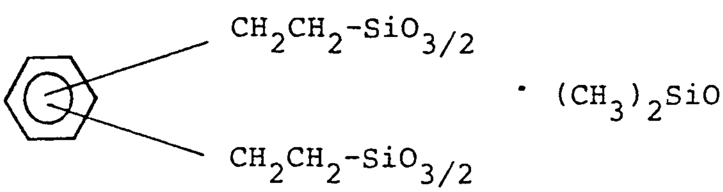

in Form eines weißen, teilweise stückigen Feststoffs erhalten.

| Elementaranalysen: | % C | % H | % Si |
|---|---|---|---|
| Theorie: | 46,41 | 5,84 | 27,13 |
| Gefunden: | 45,22 | 5,82 | 26,71 |

Beispiel 5

Ausgehend von 50 g des in Beispiel 4 verwendeten Phenylengruppen-haltigen Organosilans, 113,8 g

$Ti(O\text{-}iC_3H_7)_4$, 100 ml Isopropanol und 50 ml Wasser wurden analog zu Beispiel 4 63,3 g (99,6 % der Theorie) eines polymeren Produkts, bestehend aus Einheiten der Formel

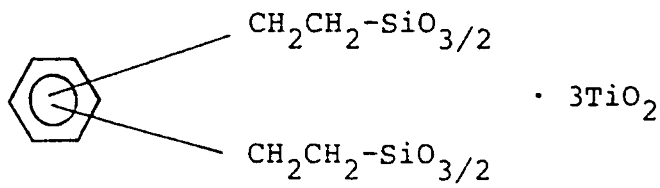

in Form eines weißen Feststoffs erhalten.

| Elementaranalysen: | % C | % H | % Si | % Ti |
|---|---|---|---|---|
| Theorie: | 25,23 | 2,54 | 11,80 | 30,18 |
| Gefunden: | 24,60 | 2,67 | 10,95 | 29,49 |

Beispiel 6

Ausgehend von 60 g eines Phenylengruppen-haltigen Organosilans, bestehend zu rund 100 % aus dem ortho- und meta-Isomerengemisch (60 Gew.% / Gew.%) des Ethoxisilans

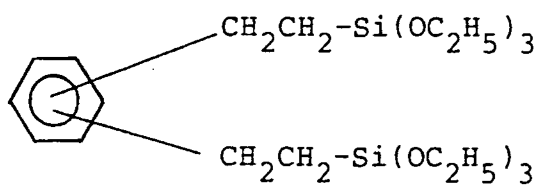

42,8 g $Zr(O\text{-}nC_3H_7)_4$, 100 ml Ethanol und 40 ml Wasser wurden analog zu Beispiel 4 46,3 g (98,4 % der Theorie) eines polymeren Produkts, bestehend aus Einheiten der Formel

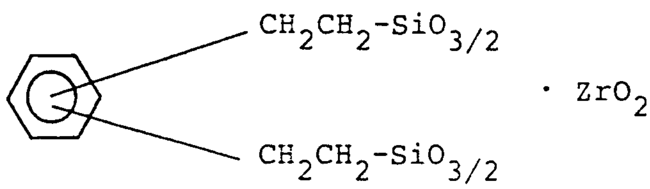

in Form eines weißen Feststoffs erhalten.

| Elementaranalysen: | % C | % H | % Si | % Zr |
|---|---|---|---|---|
| Theorie: | 33,40 | 3,36 | 15,62 | 25,37 |
| Gefunden: | 32,95 | 3,87 | 15,20 | 24,29 |

Beispiel 7

Ausgehend von 100 g des in Beispiel 6 verwendeten Organosilans, 44,1 g $(H_5C_2)Al(O\text{-}C_4H_9)_2$, 100 ml Ethanol und 40 ml $H_2O$ wurden analog zu Beispiel 4 67,0 g (99,7 % der Theorie) eines polymeren Produkts, bestehend aus Einheiten der Formel

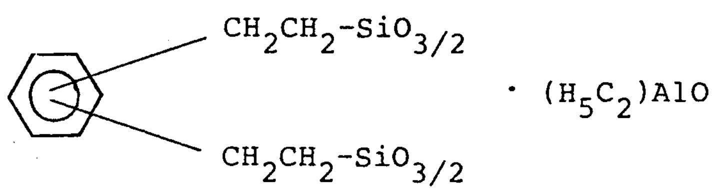

erhalten.

| Elementaranalysen: | % C | % H | % Si | % Al |
|---|---|---|---|---|
| Theorie: | 46,73 | 5,56 | 18,21 | 8,75 |
| Gefunden: | 45,68 | 5,50 | 17,36 | 8,44 |

**Patentansprüche**

1. Phenylengruppen-haltige Organopolysiloxane dadurch gekennzeichnet, daß sie gleiche oder verschiedene Einheiten der allgemeinen Formel

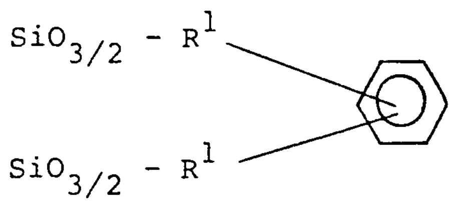

(1)

enthalten, in der jeweils alle 3 denkbaren Isomeren in bezug auf die Stellung der $SiO_{3/2}$-$R^1$-Substituenten an der Phenylengruppe nebeneinander vorliegen können, wobei die Brückenglieder $R^1$ für die Gruppen $-CH_2-CH_2-$oder

$$CH_3-CH<$$

stehen und gleich oder verschieden sein können, und die freien Valenzen der Sauerstoffatome durch Siliciumatome weiterer Gruppen der Formel (1) und/oder durch vernetzende Brückenglieder
$SiO_{4/2}$ oder $R'SiO_{3/2}$ oder $R'_2\ SiO_{2/2}$,
$TiO_{4/2}$ oder $R'TiO_{3/2}$ oder $R'_2\ TiO_{2/2}$,
$ZrO_{4/2}$ oder $R'ZrO_{3/2}$ oder $R'_2\ ZrO_{2/2}$,
$AlO_{3/2}$ oder $R'AlO_{2/2}$
wobei R' eine Methyl- oder Ethylgruppe ist, abgesättigt sind und das Verhältnis der Summe der Si-Atome in Formel (1) zu den Brückenatomen Silicium, Titan, Zirkonium und Aluminium 1 : 0 bis 1 : 15 betragen kann.

**2.** Verfahren zur Herstellung von Verbindungen gemäß Anspruch 1, dadurch gekennzeichnet, daß ein Silan der allgemeinen Formel

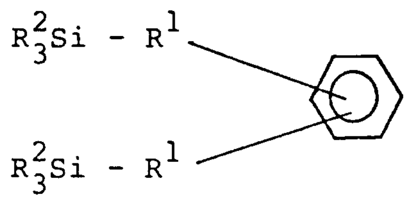

(2)

in der die Brückenglieder $R^1$ für die Gruppen $-CH_2-CH_2-$ oder

$$CH_3-CH<$$

stehen und gleich oder verschieden sein können, und die Substituenten $R^2$ für einen linearen oder verzweigten Alkoxirest mit 1 bis 3 C-Atomen oder für Chlorid stehen und gleich oder verschieden sein können, gegebenenfalls nach Zusatz eines Lösungsmittels und/oder einer Vernetzervorstufe der allgemeinen Formel

$$MeR^3_{2-4}R'_{0-2} \quad \text{bzw.} \quad MeR^3_{2-3}R'_{0-1}$$

wobei Me = Si, Ti, Zr bzw. Al
$R^3$ für einen linearen oder verzweigten Alkoxirest mit 1 bis 5 C-Atomen oder Für Chlorid steht und
R' eine Methyl- oder Ethylgruppe ist, mit stöchiometrischen oder überschüssigen Mengen Wasser hydrolysiert und polykondensiert, das Produkt gegebenenfalls nach Zusatz weiteren Lösungsmittels von der flüssigen Phase abgetrennt, gegebenenfalls gewaschen, gegebenenfalls unter Schutzgasatmosphäre oder unter Verwendung von Vakuum bis zu einer Temperatur von 200° C getrocknet, anschließend gegebenenfalls 1 Stunde bis zu 5 Tagen bei Temperaturen von 100 - 400° C, gegebenenfalls an der Luft oder unter Schutzgas, bei Normaldruck, unter Vakuum oder Überdruck getempert sowie gegebenenfalls gemahlen und klassifiziert wird.

**3.** Verfahren gemäß Anspruch 2, dadurch gekennzeichnet, daß als Lösungsmittel Methanol, Ethanol, n- und i-Propanol, n- und i-Butanol, n-Pentanol, Toluol, Xylol, chlorierte Kohlenwasserstoffe, Aceton und Dialkylether verwendet werden.

**4.** Verwendung der gemäß Anspruch 2 erhältlichen Phenylengruppen-haltigen Organopolysiloxane nach Anspruch 1 zur Synthese von Wirkstoff-Trägern durch Substitution mindestens eines der ringständigen Wasserstoffatome durch Trägerfunktion vermittelnde Substituenten nach üblichen Methoden der organischen Chemie.

## Claims

1. Organopolysiloxanes containing phenylene groups, characterised in that they contain identical or different units corresponding to the following general formula

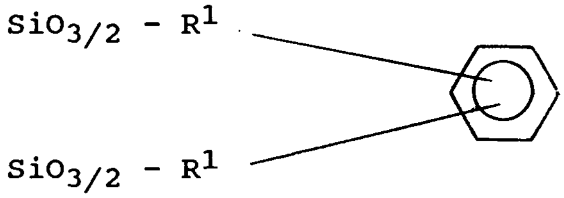

(1)

in which all 3 conceivable isomers with respect to the position of the $SiO_{3/2}$-$R^1$ substituents on the phenylene group may be present side by side and in which the bridging members $R^1$ stand for the groups -$CH_2$-$CH_2$- or

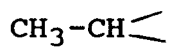

and may be identical or different, and the free valencies of the oxygen atoms are saturated by silicon atoms of other groups corresponding to formula (1) and/or by cross-linking bridging members
$SiO_{4/2}$ or $R'SiO_{3/2}$ or $R'_2$ $SiO_{2/2}$,
$TiO_{4/2}$ or $R'TiO_{3/2}$ or $R'_2$ $TiO_{2/2}$,
$ZrO'_2{}_{4/2}$ or $R'ZrO_{3/2}$ or $R'_2$ $ZrO_{2/2}$,
$AlO_{3/2}$ or $R'AlO_{2/2}$,
wherein R' denotes a methyl or ethyl group, and the ratio of the sum of Si-atoms in Formula (1) to the bridging atoms, silicon, titanium, zirconium and aluminium may be from 1 : 0 to 1 : 15.

2. A process for the preparation of compounds according to Claim 1, characterised in that a silane corresponding to the following general formula

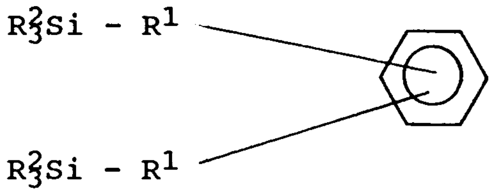

(2)

in which the bridging members $R^1$ stand for the groups $CH_2$-$CH_2$ or

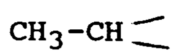

and may be identical or different and the substituents $R^2$ stand for a linear or branched alkoxy group having 1 to 3 carbon atoms or for chloride and may be identical or different, is hydrolyzed with stoichiometric or excess quantities of water and polycondensed, optionally after the addition of a solvent and/or a cross-linking precursor corresponding to the general formula

$$MeR^3_{2-4}R'_{0-2} \quad \text{or} \quad MeR^3_{2-3}R'_{0-1}$$

wherein Me = Si, Ti, Zr or Al,
$R^3$ stands for a linear or branched alkoxy group having 1 to 5 carbon atoms or for chloride and

R' stands for a methyl or ethyl group, the product obtained is separated from the liquid phase, optionally after the additon of further solvent, and is optionally washed, optionally dried at a temperature of up to 200 °C under a protective gas atmosphere or employing a vacuum, and is then optionally tempered for a period of from 1 hour to 5 days at temperatures of from 100 - 400 °C, optionally in air or under a protective gas, at normal pressure, under a vacuum or under excess pressure, and is optionally ground and classified.

**3.** A process according to Claim 2, characterised in that the solvents used are methanol, ethanol, n-and i-propanol, n- and i-butanol, n-pentanol, toluene, xylene, chlorinated hydrocarbons, acetone and dialkyl ethers.

**4.** Use according to Claim 1 of the phenylene group-containing organopolysiloxanes obtainable according to Claim 2 for the synthesis of carriers of active substances by the substitution of at least one of the ring hydrogen atoms by substituents transmitting a carrier function by conventional methods of organic chemistry.

**Revendications**

**1.** Organopolysiloxanes portant des groupes phénylène caractérisés en ce qu'ils contiennent des groupes identiques ou différents de formule générale

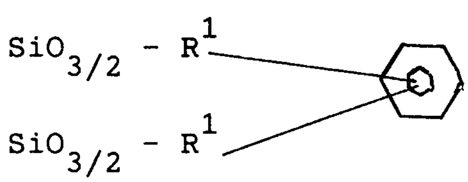 (1)

dans laquelle chacun des trois isomères possibles en relation avec la position des substituants $SiO_{3/2}$-$R^1$ du groupe phénylène, peut se présenter l'un à côté de l'autre, pour laquelle les éléments de jonction $R^1$ représentent des groupes $-CH_2$- $CH_2$- ou

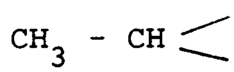

et peuvent être les mêmes ou différents et les valences de l'atome d'oxygène sont saturées par d'autres groupes d'atome de silicium de formule (1) et/ou par des éléments de jonction réticulants
$SiO_{4/2}$ ou $R'SiO_{3/2}$ ou $R'_2$ $SiO_{2/2}$
$TiO_{4/2}$ ou $R'TiO_{3/2}$ ou $R'_2$ $TiO_{2/2}$
$ZrO_{4/2}$ ou $R'ZrO_{3/2}$ ou $R'_2$ $ZrO_{2/2}$
$AlO_{3/2}$ ou $R'AlO_{2/2}$
dans lesquels R' est un groupe méthyle ou un groupe éthyle, en ce qu'ils sont saturés
et en ce que le rapport de la somme des atomes de silicium dans la formule (1) aux atomes de jonction de Silicium, Titane, Zirconium et Aluminium peut s'élever de 1 : 0 à 1 : 15.

**2.** Procédé d'obtention des composés selon la revendication 1, caractérisé en ce que l'on hydrolyse ou polycondense avec des quantités d'eau stoechiométriques ou en excès un silane de formule

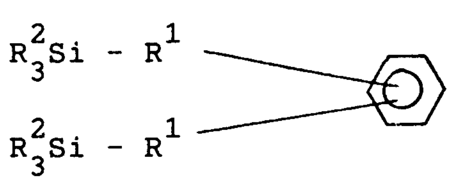 (2)

dans laquelle les éléments de jonction $R^1$ représentent des groupes $-CH_2-CH_2$ ou

$$CH_3-CH<$$

et peuvent être identiques ou différents et les substituants $R^2$ représentent un reste alcoxy linéaire ou ramifié ayant de 1 à 3 atomes de Carbone ou un chlorure et peuvent être identiques ou différents, éventuellement par addition d'un solvant et/ou d'un stade préliminaire d'un agent réticulant de formule générale

$$MeR^3_{2-4}R'_{0-2} \quad \text{ou} \quad MeR^3_{2-3}R'_{0-1}$$

dans laquelle Me est du silicium, du titane, du zirconium ou de l'aluminium.
$R^3$ représente un reste alcoxy linéaire ou ramifié ayant de 1 à 5 atomes de carbone ou un chlorure et R' est un groupe méthyle ou éthyle,
en ce que l'on sépare le produit de la phase liquide éventuellement par addition d'une nouvelle quantité de solvant,
en ce qu'on lave éventuellement et sèche éventuellement sous atmosphère de gaz protecteur ou en employant le vide à une température jusqu'à 200° C, qu'ensuite éventuellement on le recuit pendant une heure à 5 jours à des températures de 100 à 400° C, éventuellement à l'air ou sous gaz protecteur, à pression normale, sous vide ou sous surpression ainsi qu'on le broye éventuellement et le classifie.

**3.** Procédé selon la revendication 2, caractérisé en ce que l'on utilise comme solvant le méthanol, l'éthanol, le n-propanol, l'isopropanol, le n-butanol et l'isobutanol, le n-pentanol, le toluène, le xylène, les hydrocarbures chlorés, l'acétone et les éthers dialcoyliques.

**4.** Utilisation des organopolysiloxanes porteurs de groupes phénylène, obtenus selon la revendication 1, pour la synthèse de vecteurs d'éléments actifs par substitution d'au moins un des substituants intermédiaires des atomes d'hydrogène portés par le noyau, par une fonction vecteur, selon les méthodes usuelles de la chimie organique.